Europäisches Patentamt

European Patent Office

Office européen des brevets

(11) Publication number: **0 315 306**
**A1**

# (12) EUROPEAN PATENT APPLICATION

(21) Application number: **88308556.5**

(51) Int. Cl.⁴: **A61M 5/32**

(22) Date of filing: **16.09.88**

(30) Priority: **26.09.87 GB 8722667**
**21.12.87 GB 8729743**

(43) Date of publication of application:
**10.05.89 Bulletin 89/19**

(84) Designated Contracting States:
**AT BE CH DE ES FR GB GR IT LI LU NL SE**

(71) Applicant: **Jensen, Hans Skovgard**
**Borgergade 28 Magleby**
**DK-4672 Klippinge(DK)**

(72) Inventor: **Jensen, Hans Skovgard**
**Borgergade 28 Magleby**
**DK-4672 Klippinge(DK)**

(74) Representative: **Jukes, Herbert Lewis**
**Swann, Elt & Company 31 Beaumont Street**
**Oxford OX1 2NP(GB)**

(54) **Syringes and probe needles.**

(57) A syringe (21) (e.g. a hyperdermic syringe) comprises:

a container (22) for syringeable material;

probe means (24) comprised by said container (22), said probe means (24) having a first end portion (24A) for passage of syringeable material;

piston means (25) moveable in said container (22), for allowing said passage of syringeable material;

receptacle means (25A,25B) for sheathing at least said first end portion (24A) of said probe means (24); and

latch means (24C,25C) for latching at least said sheathed first end portion (24A) of said probe means (24) so as to resist re-use of at least said first end portion (24A) of said probe means (24).

FIG. 3

EP 0 315 306 A1

## SYRINGES AND PROBE NEEDLES

Syringes are used to inject materials into and/or onto receptors, or to withdraw materials from donors. Some receptors are portions of animals, humans, apparatus, devices, or machines. Some donors are portions of animals, humans, apparatus, devices, or machines. A receptor or donor may contaminate a syringe or any component thereof, e.g. a probe needle (for instance a hyperdermic or injection needle) optionally comprised by a syringe. Thus, when there is risk of contamination of a syringe or any component thereof, the syringe or component should not be re-used unless suitably decontaminated before re-use. Some syringes or components thereof are not suitable for being decontaminated, e.g. when a decontamination method may damage or destroy the syringe or a component thereof. If decontamination is not available or cannot be relied upon, there may be necessity to render the syringe or component not suitable for re-use. This necessity is a major problem, e.g. when a syringe or probe needle is available to drug and/or potential AIDS sufferers, or when a safe disposal system is not available.

It has now been found in accordance with the present invention that a syringe and/or e.g. a probe needle can be adapted to resist re-use of the syringe and/or probe needle.

A first aspect of the invention provides a syringe, comprising:
a container for material to be injected into an acceptor or for material to be withdrawn from a donor;
probe means comprised by (e.g. integral with or removeably mounted to) said container, the probe means having a first end portion for injecting said material into an acceptor or for receiving said material from a donor;
piston means moveable in the container, the piston means having a first end moveable for enabling said material to pass out of or into the container via the probe means;
receptacle means for sheathing at least the first end portion of the probe means; and
latch means for latching at least the sheathed first end portion of the probe means so as to resist re-use of at least the first end portion of the probe means.

A second aspect of the invention provides probe means adapted for the first aspect of the invention.

A third aspect of the invention provides receptacle means adapted for the first aspect of the invention.

A fourth aspect of the invention provides latch means adapted for the first aspect of the invention.

The present invention may be embodied in any suitable manner. Any suitable material(s) may constitute the syringe or any component thereof. For example, the container and/or piston means may be made of plastic(s) materials. The container may be a generally cylindrical barrel. The container may comprise at least one aperture means communicating with the probe means. Said aperture means may be comprised by a nozzle integral with or removeable from the container. The probe means may be integral with or removeable from the container. The probe means may be a probe needle or a probe tube. The probe means may comprise coupling means for coupling a second end portion of the probe means to the container. Said coupling means may comprise a hub for being mounted or seated on said nozzle. The syringe may comprise indicator means to distinguish between e.g. the first and second end portions of the probe means, e.g. indicator means comprising optional colouring may be comprised by the first or second end of the probe means.

A first kind of said receptacle means may be not a component of the container or piston means. The first kind of receptacle means may be a sheath (e.g. a cap or sleeve) having an inlet for receiving at least the first end portion of the probe means. A second kind of said receptacle means may be a component of the piston means. The second kind of receptacle means may be a chamber external and/or internal of the piston means. The chamber may have an inlet communicating with the outer end of the piston means so as to receive at least the first end portion of the probe means. A said inlet may be configured (e.g. tapered) to aid said sheathing.

Said latching may be constituted in any suitable manner. The latching may comprise surfaces abutting, engaging, fitting, or mating against each other. The act of latching may comprise motion of rotation and/or of translation. Latch means may, or may not, comprise component(s) or conformation(s) of the container, probe means, receptacle means, or other means. A first kind of latch means may be formed in said receptacle means by reaction of said receptacle means to receiving at least the first end portion of the probe means. A second kind of latch means may be formed in said piston means by reaction of the piston means to said receptacle means receiving at least the first end portion of the probe means. A third kind of latch means may comprise at least one preformed component. For example, the container and/or probe means may comprise first latch component means (e.g. a barb, clip, or recess), and/or the piston means may comprise second latch component means (e.g. a barb, clip, or recess). A fourth kind of latch means may comprise plug means for plugging an inlet to an optional chamber in the piston means, the probe means having previously been entirely sheathed in the chamber via said inlet, so that the plugged inlet entraps the sheathed probe means. It will be appreciated that when at least the sheathed first end portion of the probe means is latched, a second end portion of the probe means may be flush with an end of the container or piston means, or be within the container or piston means, or project from that end ( e.g. to provide a grip for a person's fingers during the act of sheathing). Contact is not required between a person and the first end portion of the probe means, to enable sheathing and latching of the first end portion of the probe means.

In carrying out the present invention, further resistance to re-use of at least the first end portion of the probe means may be provided. For example, the container and/or the probe means may be provided with predetermined breakability that will enable the container and/or the probe means to be broken so as to resist re-use of the container and/or probe means. When the container comprises the nozzle and the probe means comprises the hub, the hub may have said breakability, e.g. constituted by at least one hub region of transverse weakness, for instance a grooved hub region. The at least one breakable hub region may be outside the receptacle means for sheathing at least the first end of the probe means. Additionally or alternatively, when the container comprises the nozzle and the probe means comprises the hub, the nozzle may have said breakability, e.g. constituted by at least one nozzle region of transverse weakness, for instance a groved nozzle region. The at least one breakable nozzle region may be outside the hub.

It should be noted that the nozzle and/or the hub may comprise latch means for latching the hub and nozzle together. This latching may be constituted in any suitable manner, e.g. in similar manner as the aforementioned latching, cf. the first, second, third, and fourth latch means.

Guarding against accidental pricking or the like by the probe means may be constituted by guard means , e.g. by at least one guard surface, for instance constituted by a guard flange comprised by and surrounding the inlet end of the receptacle means for sheathing at least the first end portion of the probe means. It may be convenient to provide the container or any other portion of present invention with at least one guard surface.

In the accompanying drawings, which are by way of example of the present invention:

Fig. 1 shows a syringe with an integral probe needle, before or during use.

Fig. 2 shows the syringe of Fig. 1 when the tip of the probe needle is sheathed and captive within a protective sleeve or sheath.

Fig. 3 shows a syringe with a removeable probe needle, before or during use.

Fig. 4 shows an alternative manner of sheathing and holding captive the probe needle of Fig. 3.

Fig. 5 shows the syringe of Fig. 3 when the tip of the probe needle is sheathed and captive within a portion of the syringe's piston.

Figs. 6,7 show an alternative construction for detail X of Fig. 5.

Fig. 8 shows an alternative construction for detail X of Fig. 5.

Fig. 9 shows an alternative construction for detail X of Fig. 5.

Fig. 10 shows an alternative construction for detail X of Fig. 5.

Fig. 11 shows an alternative construction for detail X of Fig. 5.

Fig. 12 shows an alternative piston for use with the syringe of Fig. 5.

Figs. 13,14 show predetermined hub breakability Z.

Figs. 15,16 show predetermined nozzle breakability Y.

Fig. 17 shows a sheathing cap with a guard flange.

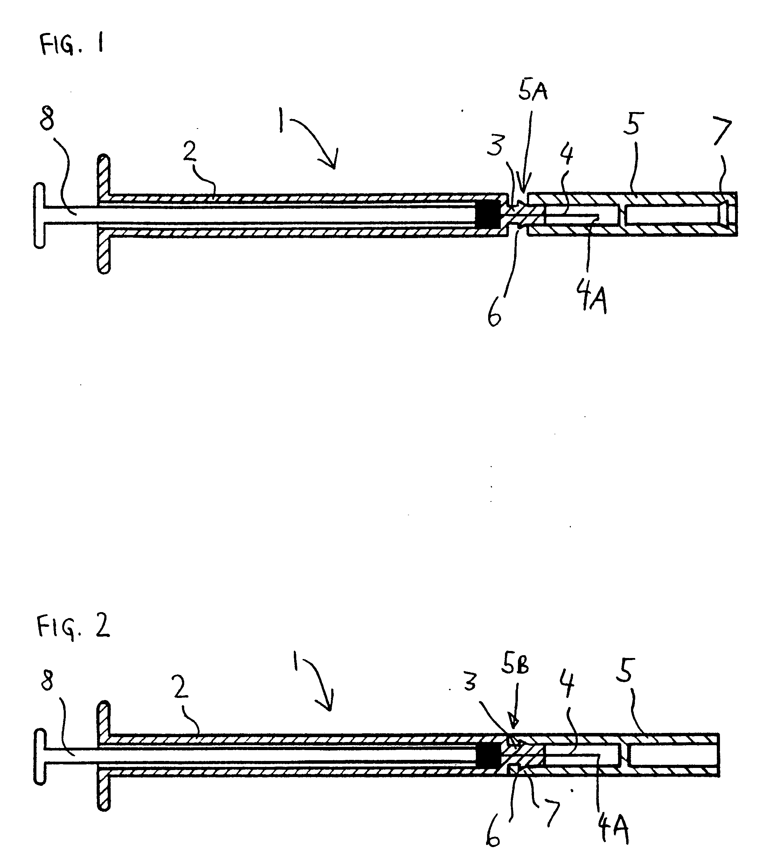

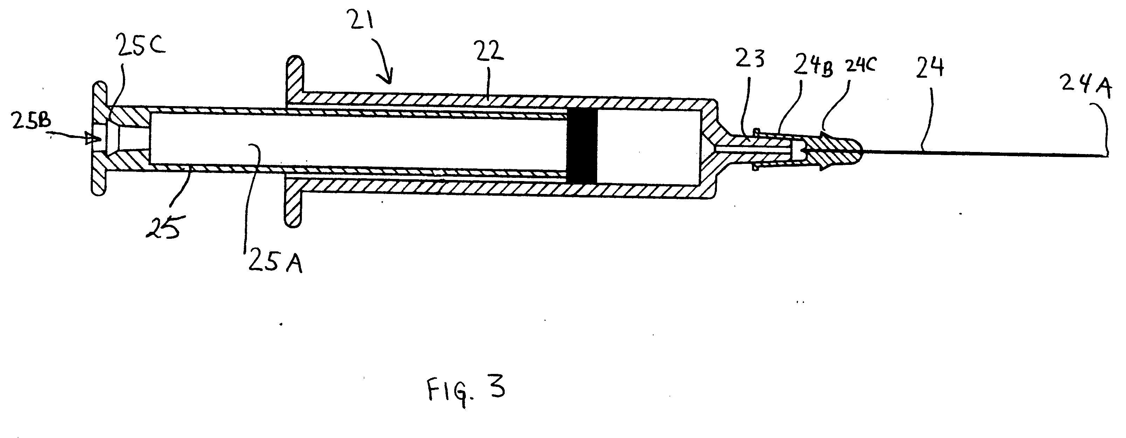

In Figs. 1,2, syringe 1 has a cylindrical barrel 2 having a barbed nozzle 3 integral with barrel 2. A probe needle 4 (e.g. a hyperdermic needle) is integral with nozzle 3 but may be a coupling hyperdermic needle as an alternative construction for engaging nozzle 3 (e.g. 24 in Fig. 3). Probe needle 4 is covered by a protective sheathing sleeve 5 having two modes of use 5A (Fig. 1) and 5B (Fig. 2). Mode 5A corresponds to storage of syringe 1 before or during use. Mode 5B corresponds to the act of sheathing and latching probe needle 4 after final use. Mode 5B is provided by urging together nozzle 3 and sleeve 5 so that a barb 6 integral with the exterior of nozzle 3, or a barb 24C of a hub 24B (Fig. 3), defines a corresponding recess 7 in the inner wall of sleeve 5, the barb 6 or 24C thereby being latched together to hold captive the sheathed tip 4A of probe needle 4 (which may be a hyperdermic needle for entering skin of an animal or human). The sheathing and latching entrap the tip 4A so as to render it unretrievable and untouchable. Barrel 2 contains piston 8.

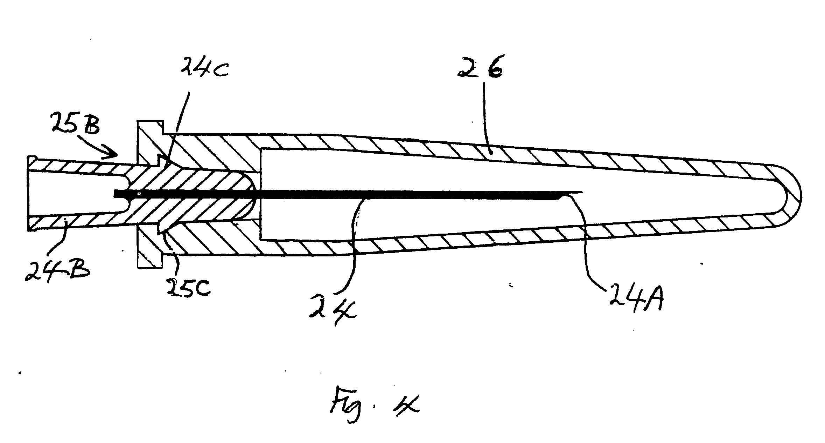

Fig. 4 shows a sheathing cap 26 for sheathing and latching to hub 24B, in a similar manner to the sheathing and latching in Fig. 2.

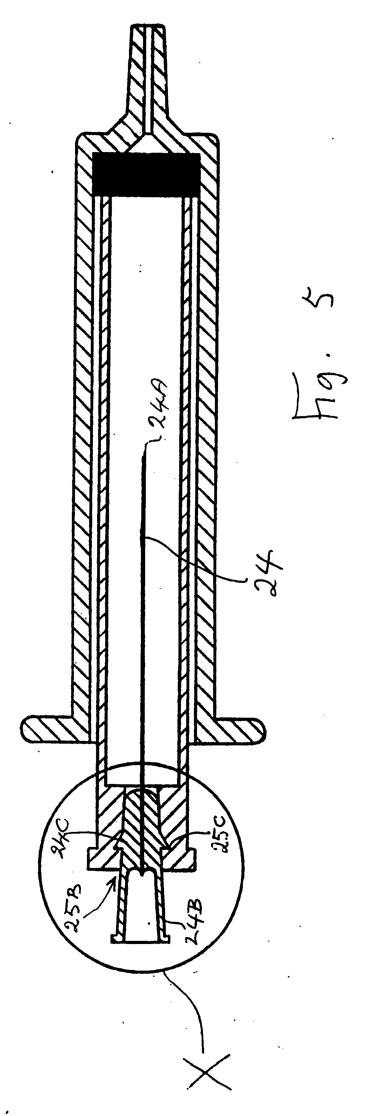

In Figs. 3, 5, a syringe 21 has a cylindrical barrel 22 having a nozzle 23 integral with barrel 22. A coupling probe needle 24 (e.g. a hyperdermic needle) has a coupling hub 24B for being mounted or seated on nozzle 23. Hub 24B has a projecting barb 24C integral with hub 24B. Barrel 22 contains a piston 25 having an internal chamber 25A with an inlet 25B at the outer end of piston 25.

Fig. 5 shows syringe 21 after use and when probe needle 24 has been removed from nozzle 23 and inserted into chamber 25A via inlet 25B. This insertion causes barb 24C to define a corresponding recess 25C in the inner wall of inlet 25B, the coupling hub 24B and recess 25C thereby being latched together to hold captive the sheathed tip 24A of probe needle 24 (which may be a hyperdermic needle for entering the skin of an animal or human). The captured sheathed tip 24A is unretrievable and untouchable.

Barb 6 (Figs. 1, 2) or barb 24C (Figs. 3, 4, 5) may be fastener components preformed before manufacture of probe needle 4 or 24. Furthermore, recess 7 (Figs. 1, 2) or recess 25C (Figs. 4, 5) may be fastener components preformed before manufacture of sleeve 5 or piston 25. Some examples of fastener components are described later below. Instead of providing nozzle 3 (Figs. 1, 2) integral with probe needle 4, Figs. 1, 2 may be modified by replacing nozzle 3 and probe needle 4 with a nozzle 23 and probe needle 24 whose barb 24C will inter-engage the sheathing sleeve 5 in the manner of modes 5A, 5B. Needle 24 may be dismounted from nozzle 23, with the needle's tip 24A sheathed and latched in sleeve 5, so as to render the tip 24A unretrievable and untouchable. In Fig. 4, the tip 24A is sheathed and latched in the sheathing cap 26.

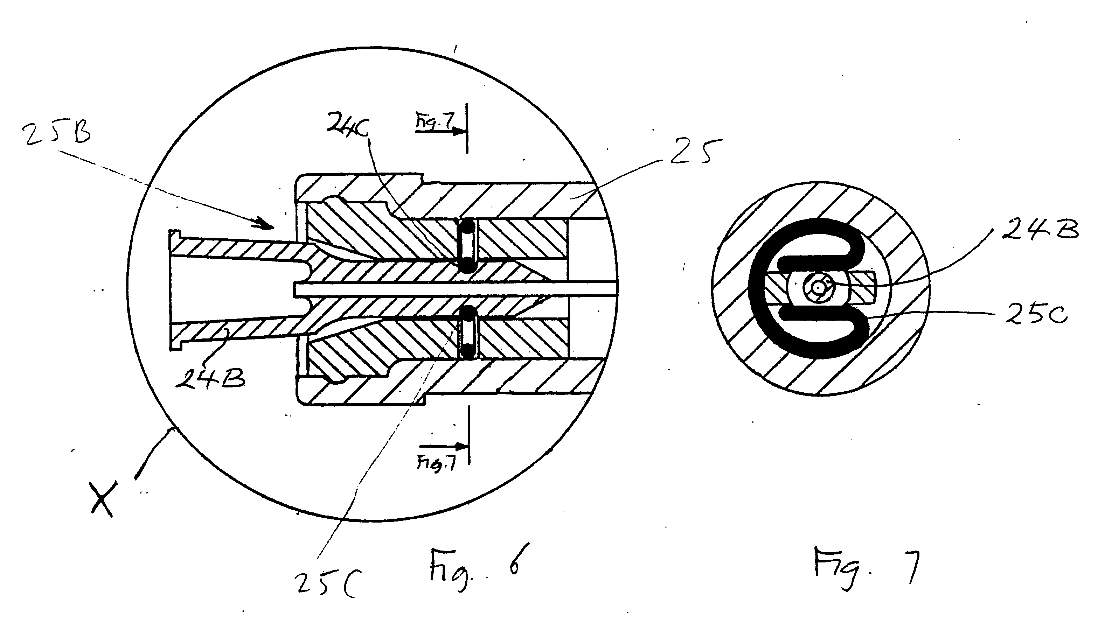

In Figs. 6, 7, alternative detail X comprises a piston inlet 25B whose inner wall contains a fastener component 25C that is a spring clip for entering a preformed recess 24C in coupling hub 24B, thereby latching together hub 24B and inlet 25B so as to hold captive the sheathed tip 24A of probe needle 24.

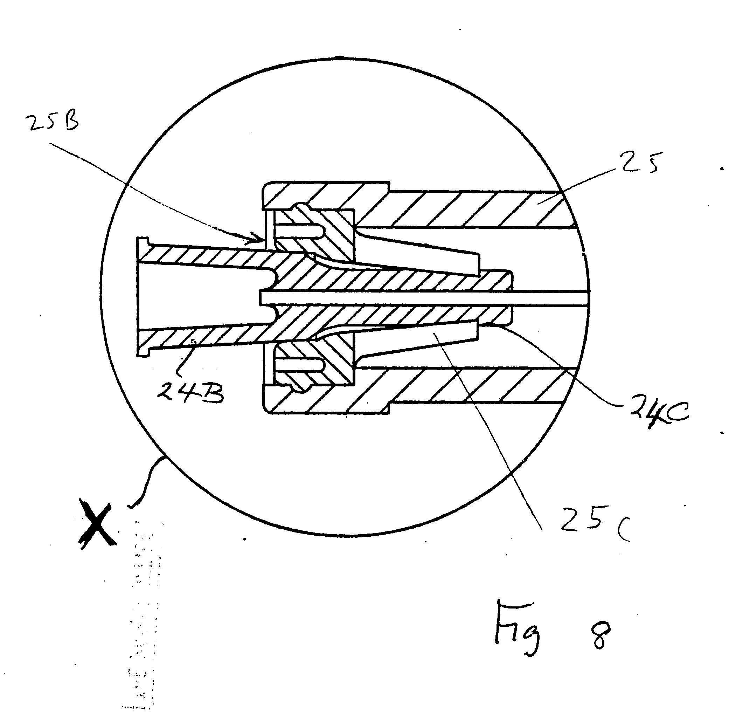

In Fig. 8, alternative detail X comprises a piston inlet 25B whose inner end portion forms a coned latch 25C that is a barb for entering a recess 24C in coupling hub 24B, thereby latching together hub 24B and inlet 25B so as to hold captive the sheathed tip 24A of probe needle 24. Recess 24C may be preformed.

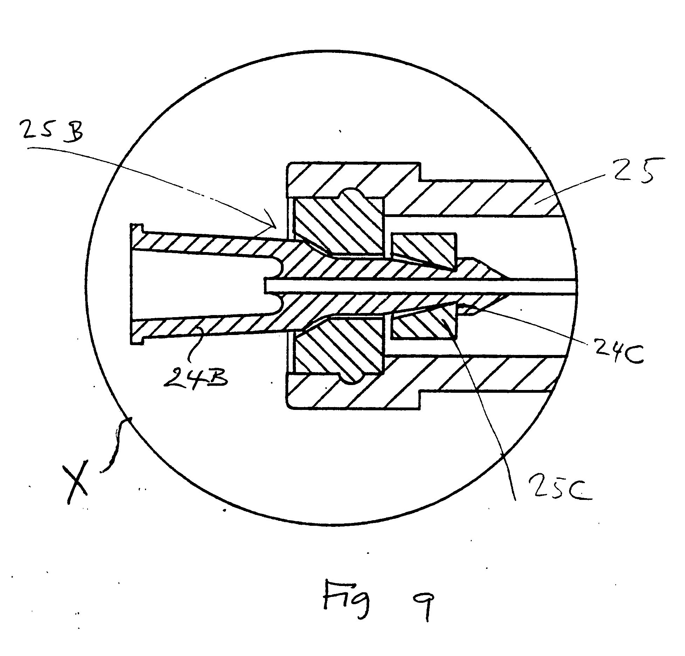

In Fig. 9, alternative detail X comprises a piston inlet 25B whose inner end portion forms a tapered wedge latch 25C that is a barb for entering a recess 24C in coupling hub 24B, thereby latching together hub 24B and inlet 25B so as to hold captive the sheathed tip 24A of probe needle 24. Recess 24C may be preformed.

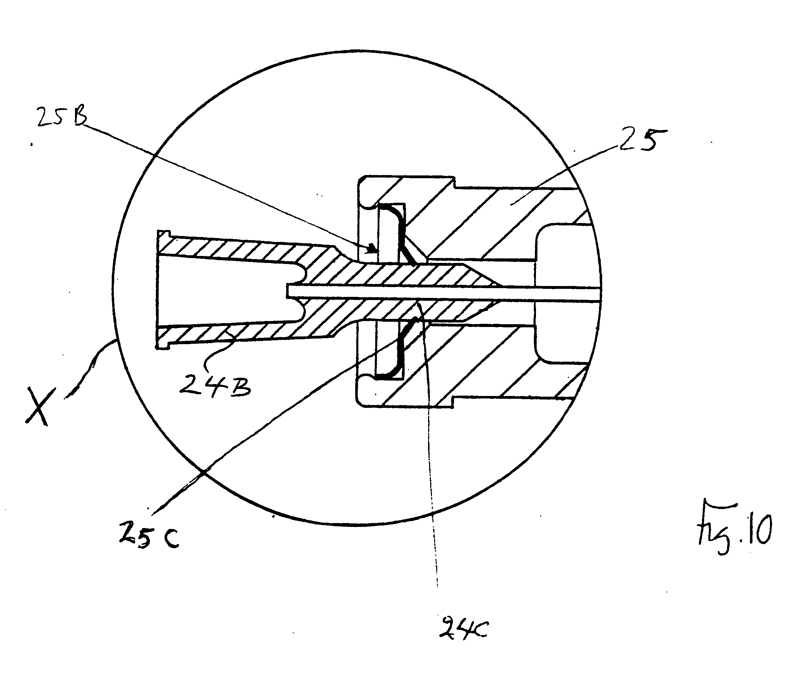

In Fig. 10, alternative detail X comprises a piston inlet 25B whose inner end contains a fastener component 25C that is a spring clip for engaging the shank 24C of coupling hub 24B, this engaging being an interference fit with the fastener component 25C optionally pressing a recess (not shown) into the exterior of shank 24C.

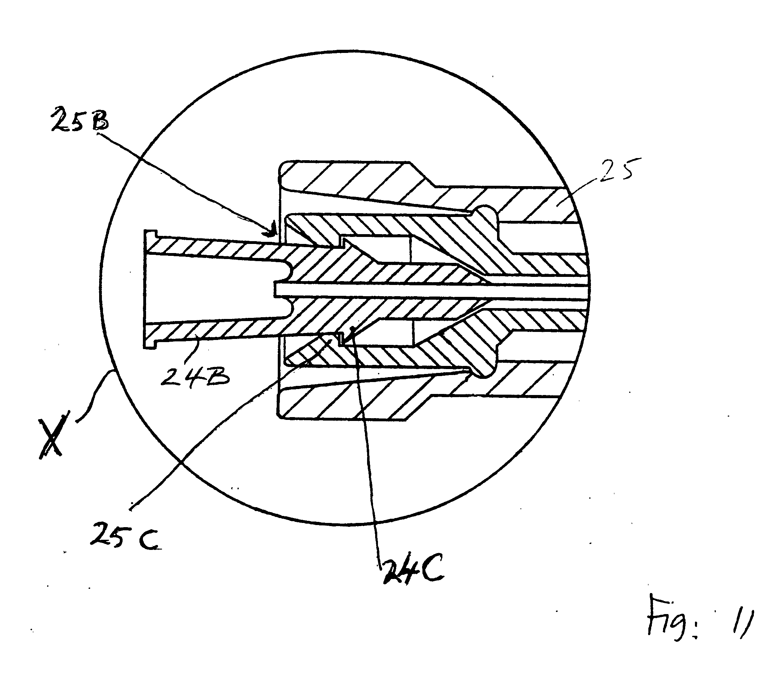

In Fig. 11, the alternative detail X comprises a piston inlet 25B whose outer portion forms a tapered mouth latch 25C that is an internal barb for abutting a corresponding barb 24C on coupling hub 24B, thereby latching together hub 24B and inlet 25B so as to hold captive the sheathed tip 24A of probe needle 24.

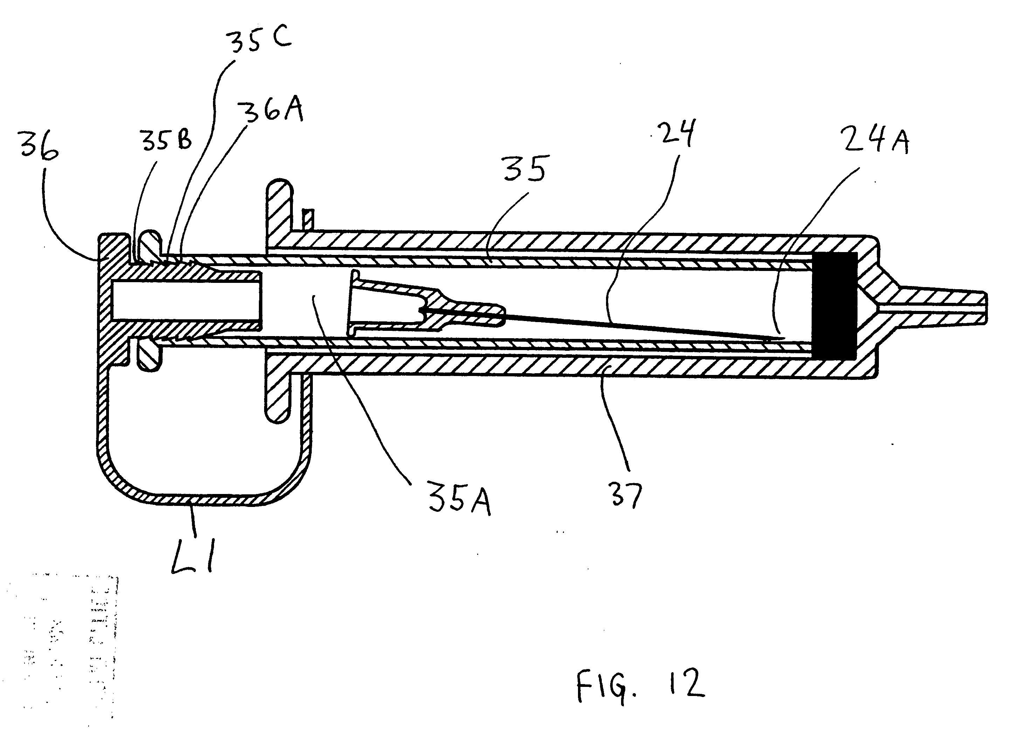

In Fig. 12, alternative piston 35 has an internal chamber 35A with an inlet 35B at the outer end of piston 35. After use of the syringe, the hyperdermic needle 24 may be introduced through inlet 35B into chamber 35A, where it may be entirely entrapped by inserting into inlet 35B a barbed plug 36 whose barbs 36A define corresponding recesses 35C in the inner wall of inlet 35B, whereby the barbs prevent withdrawal of the plug 36 from inlet 35B so as to render the hyperdermic needle 24 unretrievable and untouchable. As an alternative, the recesses 35C may be preformed.

Latch means may be provided for latching together a syringe barrel to the piston's outer end portion, and/or for latching a said plug means to the syringe barrel and/or piston, e.g. flexible link LI (Fig. l2) may connect plug 36 to barrel 37, or optionally (not shown) to the piston 35's outer portion.

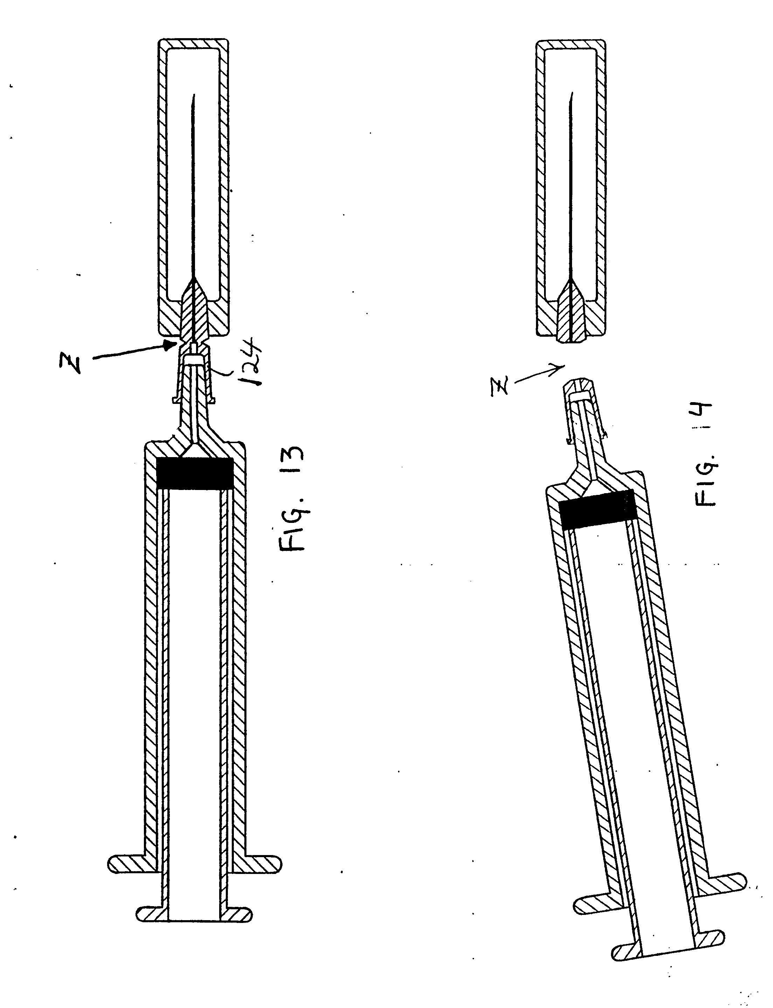

In Figs. 13,14, hub region Z is a grooved portion provided in the exterior of hub 124 ( corresponding to hub 24). Region Z is axially strong but transversely weak, thereby allowing suitable breakability as described earlier above.

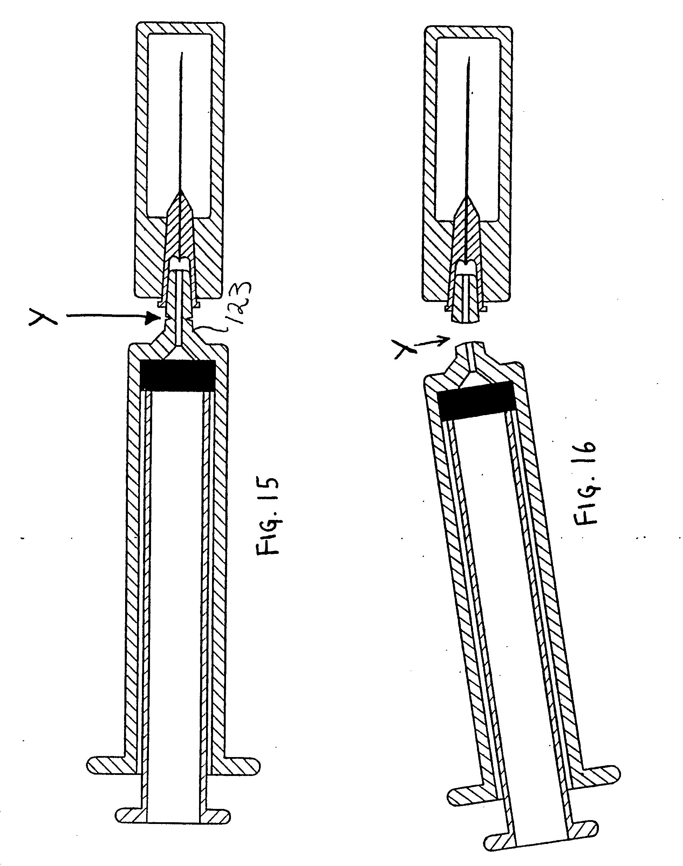

In Figs. 15,16, nozzle region Y is a grooved portion provided in the exterior of nozzle 123 ( corresponding to nozzle 23). Region Y is axially strong but transversely weak, thereby allowing suitable breakability as described earlier above.

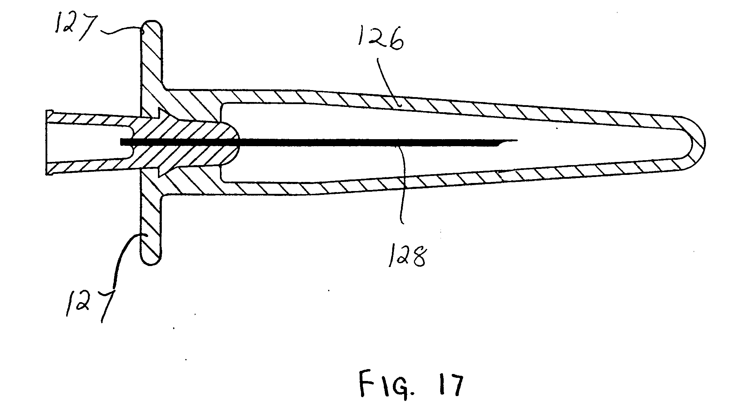

In Fig. 17, sheathing cap 126 ( corresponding to cap 26 ) has a guard flange 127 surrounding the inlet end of cap 126, to guard a person's fingers or hand against accidental pricking by the probe needle 128 during the sheathing action.

The syringes and components shown in the accompanying drawings can be modified according to the description given above before the first reference to the drawings. The present invention includes equivalents and modifications arising from all the disclosures within the present application.

## Claims

1. A syringe, characterised by comprising:
a container (2,22) for syringeable material;
probe means (4,24) comprised by said container, said probe means having a first end portion (4A,24A) for passage of said syringeable material;
piston means (8,25) moveable in said container, for allowing said passage of syringeable material;
receptacle means (5,25A,25B,35A,35B) for sheathing at least said first end portion of said probe means: and
latch means (6,7,24C,25C,35C,36A) for latching at least said sheathed first end portion of said probe means so as to resist re-use of at least said first end portion of said probe means.

2. A syringe as claimed in claim 1, characterised by said container comprises a nozzle (3,23) for communicating with said probe means.

3. A syringe as claimed in claim 1, characterised by said probe means (4) is integral with said container.

4. A syringe as claimed in claim 1, characterised by said probe means (24) is removeable from said container.

5. A syringe as claimed in claim 1, characterised by said receptacle means comprises a sheath (5) having an inlet (5B) for receiving at least said first end portion of said probe means.

6. A syringe as claimed in claim 1, characterised by said piston means comprises a said receptacle means (25A,25B,35A,35B), this receptacle means having an inlet (25B,35B) communicating with the outer end of said piston means so as to receive at least said first end portion of said probe means.

7. A syringe as claimed in any one of claims 1 to 6, characterised by said receptacle means is adapted such that at least a portion of said latch means is formed (7,25C,35C) in said receptacle means by reaction of said receptacle means to receiving at least said first end portion of said probe means.

8. A syringe as claimed in any one of claims 1 to 7, characterised by at least a portion of said latch means comprises at least one preformed latch component (6,7,24C,25C,35C,36A).

9. A syringe as claimed in any one of claims 1 to 8, characterised by said piston means comprises a said receptacle means (35A,35B); and said latch means comprises plug means (36) for plugging an inlet (35B) to that receptacle means, said probe means having previously been entirely sheathed therein, so that the plugged inlet entraps said sheathed probe means.

10. A syringe as claimed in any one of claims 1 to 9, characterised by being a hyperdermic syringe.

FIG. 1

FIG. 2

FIG. 3

EP 0 315 306 A1

EP 0 315 306 A1

24c

26

25B

24B

25C

24

24A

Fig. 4

EP 0 315 306 A1

25B
24C
25C
24B
X
24A
24

Fig. 5

25B

24C

Fig.7

25

24B

25C

24B

X

25C

Fig. 6

Fig. 7

EP 0 315 306 A1

25B

25

24B

24C

25C

X

Fig 8

EP 0 315 306 A1

Fig 9

25 B

25

24 B

25 C

X

24c

Fig. 10

EP 0 315 306 A1

25

25 B

25 C

24 B

24 C

X

Fig. 11

FIG. 12

EP 0 315 306 A1

FIG. 13

FIG. 14

EP 0 315 306 A1

Fig. 15

Fig. 16

FIG. 17

EP 0 315 306 A1

## DOCUMENTS CONSIDERED TO BE RELEVANT

EP 88308556.5

| Category | Citation of document with indication, where appropriate, of relevant passages | Relevant to claim | CLASSIFICATION OF THE APPLICATION (Int. Cl.4) |
|---|---|---|---|
| X | US - A - 4 623 336 (J.PEDICANO et al.) | 1,2,4, 5,8,10 | A 61 M 5/32 |
| Y | * Fig. 1-3; abstract; column 7, lines 49-64; column 8, lines 51-59,63-66 * | 3,6,9 | |
| Y | US - A - 4 468 223 (Y.MINAGAWA et al.) | 3 | |
| | * Fig. 1,3; column 3, lines 36-46,55,56 * | | |
| Y | US - A - 4 067 333 (F.REINHARDT et al.) | 6,9 | |
| | * Fig. 1-4 * | | |
| A | DE - A1 - 2 444 022 (G.PAGLIERE) | 6,9 | |
| | * Fig. 1,3,6 * | | TECHNICAL FIELDS SEARCHED (Int. Cl.4) |
| P,X | EP - A1 - 0 250 104 (T.A.SPENCER) | 1,2,4, 5,8,10 | A 61 M 5/00 |
| | * Fig. 12,13,17,18; column 3, lines 38-42; abstract; column 8, lines 1-22 * | | |
| P,X | EP - A2 - 0 281 421 (LUTHER MEDICAL PRODUCTS INC.) | 1,2,4, 5,8,10 | |
| | * Fig. 3-6; abstract; column 2, line 5 - column 4, line 4 * | | |

The present search report has been drawn up for all claims

| Place of search | Date of completion of the search | Examiner |
|---|---|---|
| VIENNA | 22-02-1989 | LUDW1G |